(19) Europäisches Patentamt
European Patent Office
Office européen des brevets

(11) Veröffentlichungsnummer: **0 111 717**
**B1**

(12) # EUROPÄISCHE PATENTSCHRIFT

(45) Veröffentlichungstag der Patentschrift: **04.02.87**

(51) Int. Cl.⁴: **C 07 D 241/20**, C 07 D 241/16, C 07 D 241/18

(21) Anmeldenummer: **83111081.2**

(22) Anmeldetag: **07.11.83**

(54) **Neue 2-Aminopyrazine und Verfahren zur Herstellung von 2-Aminopyrazinen und Pyrazinen.**

(30) Priorität: **15.11.82 DE 3242195**

(43) Veröffentlichungstag der Anmeldung: **27.06.84 Patentblatt 84/26**

(45) Bekanntmachung des Hinweises auf die Patenterteilung: **04.02.87 Patentblatt 87/6**

(84) Benannte Vertragsstaaten: **BE CH DE FR GB IT LI**

(56) Entgegenhaltungen:
**TETRAHEDRON, Band 30, 1974, Pergamon Press, OXFORD (GB) J. PERCHAIS et al.: "Carboacides polycyanes-V: Préparation et propriétés des sels d'aza-2 propenures polycyanes", Seiten 999-1009**
**Houben-Weyl Stickstoffverbindungen II (Amine), Seiten 16 u. 17; The Pyrazines, GB Berlin, Seite 134**

(73) Patentinhaber: **BASF Aktiengesellschaft, Carl-Bosch- Strasse 38, D-6700 Ludwigshafen (DE)**

(72) Erfinder: **Brunnmueller, Fritz, Dr., Alsenzstrasse 7, D-6703 Limburgerhof (DE)**
Erfinder: **Kroener, Michael, Dr., Eislebener Weg 8, D-6800 Mannheim 42 (DE)**

EP 0 111 717 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft neue 2-Aminopyrazine sowie ein Verfahren zur Herstellung von 2-Aminopyrazinen und Pyrazinen durch Umsetzung von α-Iminodiacetonitrilen a) mit Halogenwasserstoffen oder b) mit Alkoholen oder Thioalkoholen und Halogenwasserstoffen oder c) mit Alkoholen oder Thioalkoholen in Gegenwart von Alkali- und/oder Erdalkaliverbindungen.

Unsubstituiertes bzw. in 5-Stellung substituierte 2-Aminopyrazine werden durch Kondensation von 1,2-Dicarbonylverbindungen und dem Dihydrobromid von Aminoacetamidin in Gegenwart einer Base in Methanol bei -30°C (Pitred und Boveri, Chem. Ber. 100 (1967), 560-563) hergestellt:

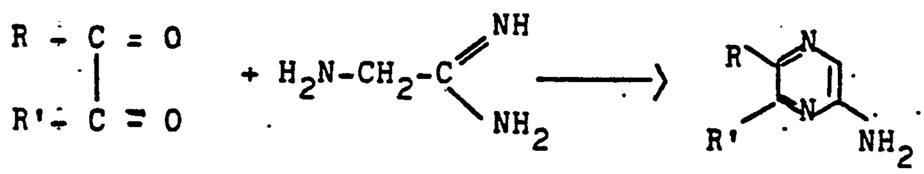

3,5-disubstituierte 2-Aminopyrazin-1-oxide erhält man (JACS 90, (1968) 2424, 2425) durch Kondensation von oximinoketonen mit α-Aminonitrilen in Eisessig bei Raumtemperatur:

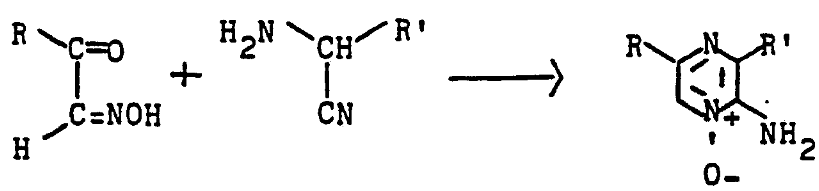

Die Oximinoverbindungen sind schwer zugänglich.

Vohra et al. (J. Org. Chem. 44, (1979), 1125-1130) beschreiben die Umsetzung des α-Iminodiacetonitrils mit Schwefelsäure und Natriumnitrit zu N-Nitroso-α-iminodiacetonitril. Die Nitrosoverbindung wird dann abfiltriert, getrocknet und zweimal aus Ethylacetat/Petrolether umkristallisiert. Erst nach dieser Reinigung wird sie mit Natriummetylatlösung unter Stickstoff bei Raumtemperatur während 16 Stunden umgesetzt. Nach Aufarbeitung des Gemischs erhält man 2-Amino-6-methoxipyrazin:

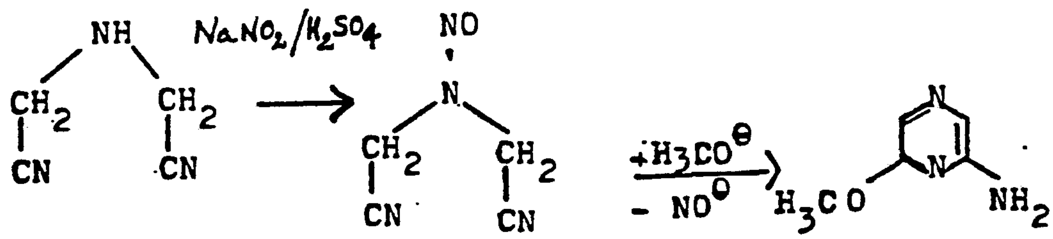


Entsprechend wird auch das 2-Amino-6-ethoxi-pyrazin hergestellt. Es wird darauf hingewiesen, daß α-Iminodiacetonitril selbst unter vorgenannten Bedingungen nicht cyclisiert, und die Lehre gegeben, daß die Herstellung des Pyrazinringes erst durch kombinierten Effekt der N-Nitroso- und Nitrilgruppen auf die Acidität der Protonen in α-Stellung zur Nitrilgruppe ermöglicht wird.

In der US-Patentschrift 3,607,910 wird gelehrt daß die Umsetzung von Iminodiacetonitril mit Methanol und wasserfreiem HCl zu Dimethyliminodiacetathydrochlorid führt. Die Bildung von 2-Aminopyrazin wird dort nicht beobachtet.

Es wurde nun gefunden, daß man Gemische von 2-Aminopyrazinen der Formel Ia

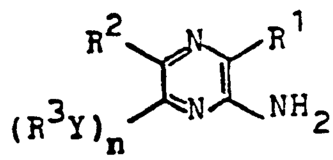

Ia

und von Pyrazinen der Formel Ib,

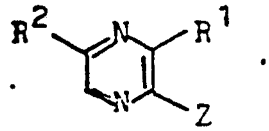

Ib

worin die einzelnen Reste $R_1$ und $R^2$ gleich oder verschieden sein können und jeweils ein Wasserstoffatom einen Alkylrest mit 1 bis 20 Kohlenstoffatomen der im Falle des Verfahrens c) durch Alkoxy- und/oder Alkylthiogruppen mit 1 bis 4 Kohlenstoffatomen im Falle des Verfahrens a) und b) durch Halogenatome substituiert sein kann einen Alkenylrest mit 2 bis 20 Kohlenstoffatomen einen Cycloalkyrest mit 5 bis 8 Kohlenstoffatomen einen Alkylarylrest oder Aralkylrest mit 7 bis 12 Kohlenstoffatomen oder einen Phenylrest bedeuten $R^3$ im Falle des Verfahrens b) einen ggf. durch Halogenatome Carbalkoxygruppen mit 2 bis 4 Kohlenstoffatomen substituierten Alkylrest mit 1 bis 20 Kohlenstoffatomen bedeutet, im Falle des Verfahrens c) einen Alkylrest mit 1 bis 8 Kohlenstoffatomen bezeichnet, Y für ein Sauerstoffatom oder Schwefelatom steht 2 ein Bromatom oder Chloratom X oder den Rest $YR^3$, worin Y und $R^3$ die vorgenannte Bedeutung besitzen, bedeutet und n 0 oder, wenn die Umsetzung c) durchgeführt wird, gegebenenfalls 1 bezeichnet, durch Cyclisierung von Dinitrilverbindungen vorteilhaft erhalt wenn man α-Iminodiacetonitrile der Formel II

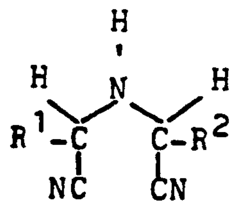

II

zur Herstellung der Verbindungen Ia und Ib,

a) für den Fall, daß Z ein Brom- oder Chloratom X bedeutet, mit Brom- oder Chlorwasserstoffen der Formel III

H - X III,

oder

b) für den Fall, daß Z ein Bromatom, Chloratom X oder den Rest $YR^3$ bedeutet, wobei $R^3$ mehr als 1 C-Atom aufweist, mit Alkoholen und/oder Thioalkoholen der Formel IV,

$HYR^3$ IV,

und Brom- oder Chlorwasserstoffen der Formel III

H - X III,

oder

c) für den Fall, daß Z den Rest $YR^3$ bedeutet, mit Alkoholen und/oder Thioalkoholen der Formel IV

$HYR^3$ IV

in Gegenwart von Alkali- und/oder Erdalkaliverbindungen

umsetzt.

Weiterhin wurden die neuen 2-Aminopyrazine der Formel Ia

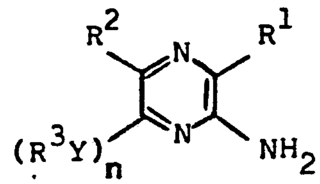

Ia,

worin die einzelnen Reste $R_1$ und $R^2$ verschieden sind und ein Wasserstoffatom einen Alkylrest mit 1 bis 20 Kohlenstoffatomen einen Alkenylrest mit 2 bis 20 Kohlenstoffatomen einen durch Alkoxy-und/ oder Alkylthiogruppen mit 1 bis I Kohlenstoffatomen oder einen durch Halogenatome substituierten Alkylrest mit 1 bis 20 Kohlenstoffatomen einen Cycloalkylrest mit 5 bis 8 Kohlenstoffatomen einen Alkylarylrest oder Aralkylrest mit 7 bis 12 Kohlenstoffatomen oder einen Phenylrest bedeuten $R^3$ einen ggf. durch Halogenatome oder Carbalkoxygruppen mit 2 bis 4 Kohlenstoffatomen substituierten Alkylrest mit 1 bis 20 Kohlenstoffatomen bezeichnet, Y für ein Sauerstoffatom oder Schwefelatom steht und n 0 oder 1 bezeichnet wobei wenn $R^1$ und $R^2$ jeweils ein Wasserstoffatom einen Methyl- Ethyl-, einen über Stickstoffatome oder Sauerstoffatome substituierten Methylrest oder einen Phenylrest bedeuten, dann n 1 bezeichnet.

Die Umsetzung kann für den Fall der Verwendung der gezeigten Ausgaugsstoffe durch die folgenden Formel wieder werden:

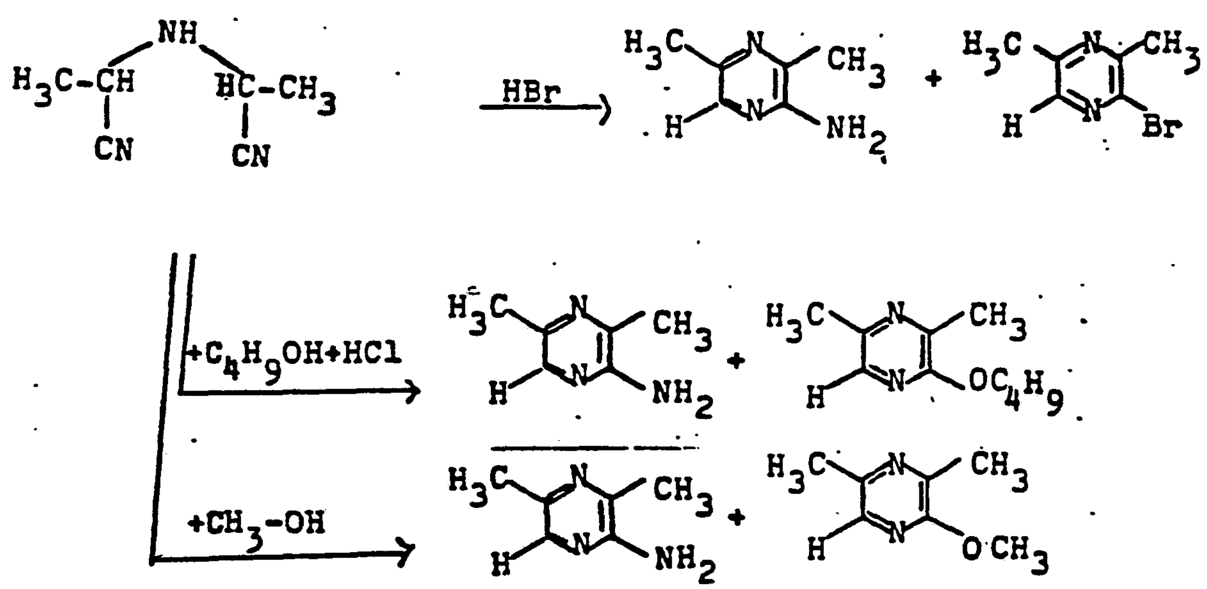


Im Hinblick auf die beschriebenen Verfahren liefert das Verfahren nach der Erfindung mit leicht zugänglichen Ausgangsstoffen auf einfacherem und wirtschaftlicherem Wege Gemische von 2-Aminopyrazinen und Pyrazinen in sehr guter Ausbeute. Im Hinblick auf die Veröffentlichung in J. org. Chem. wird bei dem erfindungsgemäßen Verfahren eine Umsetzungsstufe eingespart und ein Betrieb mit der toxischen salpetrigen Säure und ihren Nitriten vermieden. Im Hinblick auf die Veröffentlichung in den Chem. Berichten, sind die erfindungsgemäßen Ausgangsstoffe leichter zugänglich. Alle diese vorteilhaften Eigenschaften sind im Hinblick auf den Stand der Technik überraschend, man hätte erwartet, daß α-Imino-diacetonitrile 11 für eine einstufige Umsetzung zu 2-Aminopyrazinen nicht geeignet sind. Überraschend ergeben asymmetrische α-Imino-diacetonitrile II nach dem Verfahren b Endstoffe 1 mit weitgehender Regiospezifität im Hinblick auf die Reste $R^1$ und $R^2$. Unter Regiospezifität wird hier die Bevorzugung eines Isomers im Vergleich zum anderen verstanden.

Im Hinblick auf z.B. US-PS 3,607,910 war es überraschend, daß die Verfahrensweise b) bei Verwendung von in α- und in α,α'-substituierten Ausgangsstoffen II hohe Ausbeuten an den Endstoffen 1 und keine wesentlichen Mengen an substituierten Iminodiacetaten liefert.

Die Ausgangsstoffe 11 können in bekannter Weise, z.B. das α-Iminodipropionsäurenitril II durch die in DE-OS 14 93 752 beschriebene Umsetzung von Ammoniak mit Acetaldehyd und Blausäure hergestellt werden. Bevorzugt können symmetrische und die früher nicht herstellbaren asymmetrischen Ausgangsstoffe II nach der in der deutschen Patentanmeldung P 32 42 193.1 (EP-A 0 111 719) beschriebenen Arbeitsweise durch

Umsetzung von Aldehydcyanhydriuen der Formel

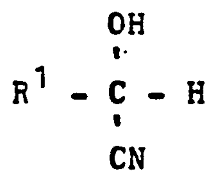

V

mit Aminonitrilen der Formel

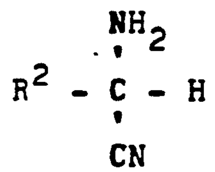

VI

worin $R^1$ und $R^2$ die vorgenannten gleichen oder unterschiedlichen Bedeutungen besitzen. Im Falle von asymmetrischen und insbesondere von symmetrischen α-Iminodiacetonitrilen II setzt man vorteilhaft in Gegenwart von niederen Alkanolen in einem Verhältnis von 5 bis 95 Gew.% Ausgangsstoff VI, bezogen auf die Gewichtsmenge an niederem Alkanol, um. Die Herstellung von α-Iminodiacetonitrilen II kann auch durch Umsetzung von Halogenacetonitrilen der Formel VII,

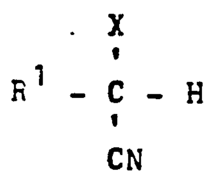

VII

worin $R^1$ und X die vorgenannte Bedeutung besitzen, mit Aminonitrilen in Gegenwart einer Hilfsbase durchgeführt werden. Die vorgenannten allgemeinen und nachgenannten bevorzugten Bedeutungen der Reste $R^1$, $R^2$ und X haben auch fur die Herstellung der Ausgangsstoffe II ihre Gültigkeit. Vorzugsweise setzt man 1 bis 2, insbesondere 1 bis 1,2 Mol Ausgangsstoff V oder 1 bis 1,5, insbesondere 1 bis 1,2 Mol Ausgaugsstoff VII je Mol Ausgangsstoff VI ein. Die Umsetzung wird zweckmäßig bei einer Temperatur von 0 bis 100, vorzugsweise 20 bis 60°C, drucklos oder unter Druck, kontinuierlich oder diskontinuierlich, vorteilhaft in Gegenwart von 1 bis 2 Mol Gesamtwasser (Reaktionswasser + zusätzliches Wasser) je Mol Ausgangsstoff VI durchgeführt. Als Hilfsbasen kommt zweckmäßig tertiäre Amine, vorteilhaft in einer Menge von 1 bis 2 Äquivalenten Amin, bezogen auf Stoff VII, in Frage. Auch organische inerte Lösungsmittel, z.B. Ether, zweckmäßig in einer Menge von 100 bis 10 000 Cew.%, bezogen auf Ausgangsstoff VII, können bei Verwendung der Stoffe VII verwendet werden.

Man kann die Ausgangsstoffe in stöchiometrischer Menge oder jede der Komponenten jeweils im Überschuß umsetzen, vorzugsweise in einem Verhältnis im Falle des Verfahrens a) von 1,5 bis 6, insbesondere 2 bis 5 Mol Ausgangsstoff III je Mol Ausgangsstoff II im Falle des Verfahrens b) von 1,5 bis 6, insbesondere 2 bis 5 Mol Ausgangsstoff III und/oder 0,1 bis 10, insbesondere 0,5 bis 3 Mol Ausgangsstoff IV je Mol Ausgangsstoff II, im Falle des Verfahrens c) von 5 bis 50, insbesondere 10 bis 25 Gew.% Ausgangsstoff II je Gewichtsmenge Ausgangsstoff IV. Bevorzugte Ausgangsstoffe II, III und IV und dementsprechend bevorzugte Endstoffe Ia und Ib sind solche, in deren Formeln $R^1$ und $R^2$ gleich oder verschieden sein können und jeweils einen Alkylrest mit 1 bis 20, vorzugsweise 1 bis 5, insbesondere 1 bis 4 Kohlenstoffatomen oder einen Alkenylrest mit 2 bis 20, vorzugsweise 2 bis 5, insbesondere 2 bis 4 Kohlenstoffatomen, (oder im Falle des Verfahrens c) einen durch Alkoxy- und/oder Alkylthiogruppen mit zweckmäßig 1 bis 4 Kohlenstoffatomen, im Falle des Verfahrens a) und b) einen durch Halogenatome, bevorzugt Chlor-, Brom-, Fluoratome substituierten Alkylrest mit 1 bis 20, vorzugsweise 1 bis 5, insbesondere 1 bis 4 Kohlenstoffatomen), einen Cycloalkylrest mit 5 bis 5 Kohlenstoffatomen, einen Alkylarylrest oder Aralkylrest mit 7 bis 12 Kohlenstoffatomen oder einen Phenylrest bedeuten, einer der Reste $R^1$ und $R^2$ auch für ein Wasserstoffatom stehen kann, $R^3$ im Falle des Verfahrens b) einen gegebenenfalls durch Halogenatome, Carbalkoxygruppen mit 2 bis 4 Kohlenstoffatomen substituierten Alkylrest mit 1 bis 20, vorzugsweise 1 bis 5, insbesondere 1 bis 4 Kohlenstoffatomen bedeutet, im Falle des Verfahrens c) einen Alkylrest mit 1 bis 5, insbesondere 1 bis 4 Köhlenstoffatomen bezeichnet, Y für

ein Sauerstoffatom oder Schwefelatom steht, Z ein Bromatom, Chloratom oder den Rest $-YR^3$, worin Y und $R^3$ die vorgenannte Bedeutung besitzen, bedeutet, und n 0 oder, wenn die Umsetzung c) durchgeführt wird, gegebenenfalls 1 bezeichnet, X für ein Bromatom oder Chloratom steht. Die vorgenannten Reste können noch durch unter den Reaktionsbedingungen inerte Gruppen z.B. Alkylgruppen, Alkoxygruppen, Alkylthiogruppen mit jeweils 1 bis 4 Kohlenstoffatomen substituiert sein. Auch Gemische von Alkoholen und Thioalkoholen können verwendet werden.

Im Falle der Umsetzung mit asymmetrischen Ausgangsstoffen II erhält man sowohl den Endstoff Ia wie den Endstoff Ib jeweils in Form seiner Isomere in 3- und 5-Stellung, z.B. der Formel

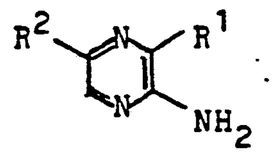 Iaa 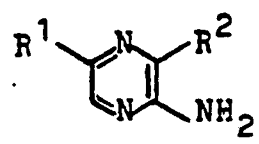 Iab

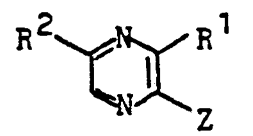 Iba 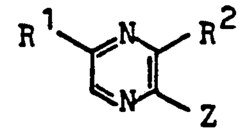 Ibb

Bei Verfahren b) ist im allgemeinen das Isomerenverhältnis von Iaa und Iab bzw. Iba und Ibb von 20 bis 4, insbesondere 15 bis 10 Mol mit kohlenstoffreicherem Substituent $R^1$ in 3-Stellung zu 1 Mol mit kohlenstoffärmerem Substituenten $R^2$ in 5-Stellung, mit $R^1$ und $R^2$ in der oben genannten Bedeutung Alkyl-, Alkenyl-, Alkylary 1-, Aralkylrest und/oder Wasserstoff.

Das Verhältnis der Endstoffe I im Gemisch beträgt im allgemeinen bei Verfahren a) von 50 bis 1, insbesondere 50 bis 2 Mol Ia je Mol Ib, bei Verfahren b (Alkoholen) von 6 bis 1, insbesondere 3 bis 2 Mol Ia je Mol Ib, bei Verfahren b (Thioalkoholen) von 50 bis 2, insbesondere 16 bis 4 Mol Ia je Mol Ib, bei Verfahren c) 100 bis 1, insbesondere 10 bis 2 Mol Ia je Mol Ib.

Es kommen z.B. als Ausgangsstoffe II in Betracht: Symmetrisch in α- und α-Stellung zu den Nitrilgruppen durch jeweils eine Methyl-, Phenyl-, Benzyl-, Cyclohexyl-, Ethyl-, Propyl-, Isopropyl-, Butyl-, sek.-Butyl-, Isobutylgruppe substituierte α-, α'-Iminodiacetonitrile; α'-unsubstituiertes Iminodiacetonitril; in der α-Stellung zu einer Nitrilgruppe unsubstituierte, in α'-Stellung zu der anderen Nitrilgruppe durch die Methyl-, Ethyl-, Propyl-, Isopropyl-, Butyl-, Isobutyl-, sek.-Butyl-, Cyclohexyl-, Benzyl-, Phenylgruppe substituierte α, α'-Iminodiacetonitrile; in α-Stellung und α'-Stellung zu den Nitrilgruppen durch vorgenannte Gruppen asymmetrisch substituierte α, α'-Iminodiacetonitrile.

Als Ausgangsstoffe III kommen Brom- und Chlorwasserstoffsäure, als Ausgangsstoffe IV bevorzugt im Falle von c) Methyl-, Ethyl-, im Falle von b) Propyl-, Butyl-, Isobutyl-, sek.-Butyl-, 2-Methylbutyl-, 2-Chloreethyl-Alkohol und entsprechende Thioalkohole, in Betracht. Bei Verfahren a) ist die Bromwasserstoffsäure besonders vorteilhaft. Die Halogenwasserstoffe können gegebenfalls auch im Gemisch mit Sulfonsäuren, insbesondere aliphatischen Sulfonsäuren mit 1 bis 4 Kohlenstoffatomen, verwendet werden.

Die Umsetzung wird zweckmäßig bei einer Temperatur von 0 bis 100, vorteilhaft im Falle der Umsetzung a) zweckmäßig von 40 bis 50°C, im Falle der Umsetzung b) zweckmäßig von 20 bis 50, bevorzugt von 30 bis 70°C, im Falle der Umsetzung c) zweckmäßig von 0 bis 50, bevorzugt 20 bis 60°C, mit Unterdruck oder Überdruck oder drucklos, diskontinuierlich oder kontinuierlich durchgeführt. Die Reaktionszeit beträgt zweckmäßig 0,1 bis 200, vorzugsweise 3 bis 45 Stunden. Man verwendet gegebenenfalls unter den Reaktionsbedingungen inerte, organische Lösungsmittel. Als Lösungsmittel kommen z.B. in Frage: aromatische Kohlenwasserstoffe, z.B. Toluol, Benzol, Ethylbenzol, o-, m-, p-Xylol; aliphatische oder cycloaliphatische Kohlenwasserstoffe, z.B. Heptan, Cyclohexan, Methylcyclohexan, Dekalin; Halogenkohlenwasserstoffe, insbesondere Chlorkohlenwasserstoffe, z.B. 1,1,2,2- oder 1,1,1,2-Tetrachlorethan, 1,2-Dichlorpropan, Methylenchlorid, Dichlorbutan, n-Propylbromid, Butylbromid, Chloroform, Tetrachlorkohlenstoff, 1,1,2-Trichlorethan, Pentachlorethan, 1,2-Dichlorethan, 1,1-Dichlorethan, n-Propylchlorid, 2-, iso-Butylchlorid, Chlorbenzol, Brombenzol, o-, p- und m-Dichlorbenzol, o-, p-, m-Dibrombenzol, o-, m-, p-Chlortoluol, 1,2,4-Trichlorbenzol (bevorzugt auch im Falle der Umsetzung a); Ether, z.B. Ethylpropylether, n-Butylethylether, Di-n-butylether, Diisobutylether, Diisoamylether, Diisopropylether, Diethylether, Dioxan (bevorzugt im Falle der Umsetzung b) mit Chlorwasserstoffsäure); Mercaptane, z.B. Butyl-, Octyl-, Dodecylmercaptan und entsprechende Gemische. Zweckmäßig verwendet man das Lösungsmittel in einer Menge von 40 bis 10 000 Gewichtsprozent, vorzugsweise von 50 bis 1 500 Gewichtsprozent, bezogen auf Ausgangsstoff II. Man kann gegebenenfalls auch den Ausgangsstoff IV selbst als Lösungsmittel nehmen; in solchen Fällen gibt man zweckmässig zu den vorgenannten Mengen an Ausgangsstoffen IV die der eingesparten Lösungsmittelmenge entsprechende Menge Ausgangsstoff IV hinzu. Im Falle von Verfahren c) werden im allgemeinen keine zusätzlichen organischen Lösungsmittel verwendet. Wegen der guten Löslichkeit von Chlorwasserstoff in Alkoholen und

Ethern kann in solchen Fällen zweckmäßig drucklos gearbeltet werden. In Dioxan-Alkohol(Thioalkohol)-Gemischen nach Verfahren b) werden besonders homogene Salzgemische gebildet, die hohe Ausbeuten an Pyrazinen Ia und Ib liefern. Im Falle von b) mit Thioalkoholen IV erhält man ein besonders hohes Molverhältnis von Ia zu Ib. Allerdings wird dann zweckmäßig bei wäßriger Aufarbeitung das Lösungsmittel gegen ein mit Wasser nicht mischbares Lösungsmittel ausgetauscht. Sehr vorteilhaft ist daher auch das Arbeiten in reinem Isobutanol, bei dem das Lösungsmittel nicht gewechselt werden braucht.

Die Umsetzung c) wird in Gegenwart einer Hilfsbase, vorteilhaft in katalytischen Mengen , zweckmäßig in einer Menge von 0,1 bis 2 vorzugsweise 0,2 bis 0,6 Äquivalenten Hilfsbase, bezogen auf ein Mol Ausgangsstoff II, durchgeführt. Die Hilfsbasen sind Erdalkaliverbindungen und insbesondere Alkaliverbindungen, vorteilhaft Alkoholate, Mercaptide, Hydroxide oder Cyanide, sowie entsprechende Gemische. Es kommen z.B. als basische Verbindungen in Frage: Kaliumhydroxid, Natriumhydroxid, Kaliumcarbonat, Natriumcarbonat, Lithiumhydroxid, Calciumhydroxid, Calciumoxid, Bariumoxid, Magnesiumhydroxid, Natriumcyanid, Natriummethanthiolat, Natriumethanthiolat, Kaliummethanthiolat, Kaliumethanthiolat, Kaliumcyanid, Natriummethylat, Hatriumpropylat, Kaliummethylat, Kaliumethylat, Kalium-n-propylat, Kaliumdiethylenglykolat.

Die Reaktion a-c) kann wie folgt durchgeführt werden: Ein Gemisch von Ausgangsstoff a) II und III, bzw. b) II, III und IV, bzw. c) II und IV und Alkaliverbindungen, gegebenenfalls zusammen mit einem organischen Lösungsmittel, wird während der Reaktionszeit bei der Reaktionstemperatur gehalten. Dann werden aus dem Reaktionsgemisch die Bndstoff ein üblicher Weise, isoliert. Endstoff Ia kann z.B. von Endstoff Ib durch fraktionierte Destillation nach der Neutralisation abgetrennt werden. Im Falle des Verfahrens a) und b) beginnt zweckmäßig die Aufarbeitung des Reaktionsgemisches müt der Neutralisation der überschüssigen Säure und der Freisetzung der Pyrazinsalze durch Alkalien, vorzugsweise Natronlauge. Nach der Extraktion der wäßrigen Phasen mit z.B. einem der vorgenannten organischen Lösungsmitteln, werden zweckmäßig die vereinigten organischen Phasen destillativ aufgearbeitet.

Als Endstoffe Ib mit Z=Halogen fallen die wegen ihrer leichteren Flüchtigkeit gut abtrennbaren 2-Halogenpyrazine der Formel

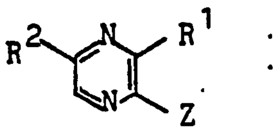

Ib

an, die durch Ammoniakeinwirkung nach bekannten Methoden auch in die 2-Aminoverbindungen überführt werden können. Verfahren a) ist für die Herstellung von unsubstituierten Endstoffen I ($R^1=R^2=H$), Verfahren b) für die Herstellung von in 3- und 5-Stellung asymmetrisch oder symmetrisch oder nur in 3-Stellung aliphatisch substituierten Enstoffen I vorteilhaft. Durch Verfahren b) mit Thioalkoholen bringt man vorteilhaft die deutlich schlechter cyclisierenden, monosubstituierten α-Iminodiacetonidtrile II ($R^1$ oder $R^2$ = H zur Reaktion. Besonders hohe Gesamtausbeuten an Pyrazinen in solchen Fällen können durch eine kombinierte Umsetzung mit Alkoholen und Thioalkoholen nach b) erzielt werden.

Verfahren c) ist insbesondere bei Verwendung von Ausgangsstoffen II mit Substituenten in 3- und 5-Stellung ($R^1=R^2$) zweckmäßig. Alkoxygruppen und/ der α-verzweigte bzw. α'-verzweigte Alkylgruppen (Verzweigung bzw. die Alkoxygruppe in 1-Stellung des Substituenten , ß-Stellung), z.B. Isopropylgruppen, enthaltende Ausgangsstoffe II werden vorteilhaft zur Herstellung von 6-substituierten Endstoffen Ia (n=l) nach Verfahren c) umgesetzt. In diesen Fällen bilden sie neben den Bndstoffen Ia (n . 0) und Ib als Hauptprodukt die Endstoffe Ia (n = 1) der Formel

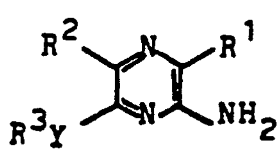

worin $R^1$, $R^2$, $R^3$ und Y die vorgenannten Bedeutungen besitzen. Eine Alkoxygruppe der Reste $R^1$ oder $R^2$ kann hierbei abgespalten werden. Beispielsweise ergeben sich folgende Umsetzungen:

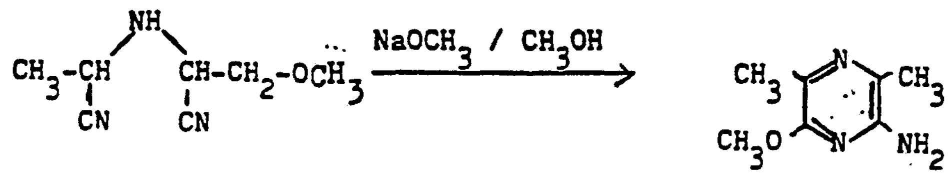


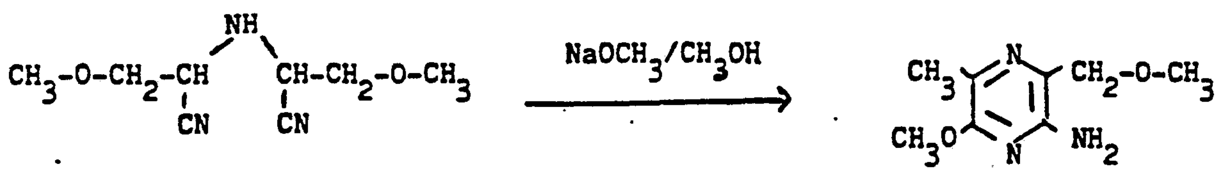


Die nach dem Verfahren der Erfindung erhältlichen 2-Aminopyrazine und Pyrazine sind wertvolle Ausgangsstoffe für die Herstellung von Farbstoffen, Fungiciden, Bactericiden, Textilhilfsmitteln und Riechstoffen. 2-Amino-3,5-dialkylpyrazine sind Vorprodukte für Folsäurederivate und 2-Halogen-pyrazine Vorprodukte für Aromastoffe, Riechstoffe und Wirkstoffe im Fflanzenschutz oder Pharmabereich. Bezüglich der Verwendung wird auf die vorgenannte deutsche Offenlegungsschrift und Ullmanns Encyklopädie der technischen Chemie, (4. Auflage), Band 7, Seite 355 und Band 20, Seite 525 und 529, verwiesen.

In einer bevorzugten Verwendungsform werden aus den so erhaltenen Endstoffen Ia, in reiner oder roher Form, nach der in der deutschen Patentanmeldung P 32 C2 266.0 (EP-A 0 111 160) beschriebenen Arbeitsweise 2-Halogen- oder 2° Cyanpyrazine der Formel

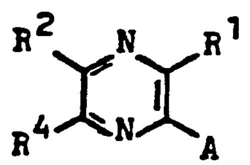

Ic,

durch Umsetzung bei einer Temperatur von -50 bis +50° C,

Ia) mit Alkalinitriten oder Alkylnitriten in Gegenwart von Wasser und/ der organischen Lösungsmitteln Ia1) mit Tetrafluorborsäure oder Ia2) Qalogenwasserstoffsäuren der Formel

H - X III,

in Form einer 10 bis 50-gewichtsprozentigen Lösung und mit Ausgangsstoff III in einer Menge von 1 bis 5 Mol Ausgangsstoff III je Mol Stoff Ia oder

Ib) mit Nitrosylhalogeniden in Gegenwart von unter den Reaktionsbedingungen inerten organischen Lösungsmitteln

und gewünschtenfalls durch Umsetzung der so erhaltenen 2-Halogenpyrazine der Formel

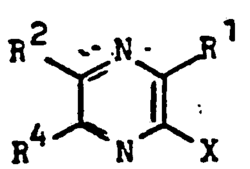

VIII,

mit Kupfercyanid und gegebenenfalls Alkali- und/oder Erdalkalicyaniden bei einer Temperatur von 50 bis 200° C hergestellt. Die Reste $R^1$, $R^2$, $R^3$, Y und X haben die vorgenannte allgemeine und bevorzugte Bedeutung, wobei A ein Halogenatom, vorzugsweise ein Chloratom, Bromatom oder Fluoratom, oder eine Cyangruppe und $R^4$ ein Wasserstoffatom oder den Rest $R^3$Y-be-deuten.

Bevorzugte Ausgangsstoffe III sind Chlor- und Bromwasserstoffsäuren. Als Nitrosylhalogenide werden

solche der Formel

O = N - X IX,

verwendet. Bevorzugte Alkalinitrite sind Natrium- und Kaliumnitrit. Bevorzugte Alkylnitrite sind solche der Formel

O = N - O - $R^5$ X,

worin $R^5$ einen Alkylrest mit 1 bis 6 Kohlenstoffatomen bedeutet, zweckmäßig Amylnitrit, Ethylnitrit, Neopentylnitrit. Verfahren 1a1 (Tetrafluorborwasserstoffsäure) wird zweckmäßig mit organischen Lösungsmitteln oder bevorzugt mit Gemischen von zusätzlichem Wasser und organischen Lösungsmitteln durchgeführt. Verfahren 1a2 kann mit organischen Lösungsmitteln und gegebenenfalls im Gemisch mit zusätzlichem Wasser, zweckmäßig in wäßrigem Medium durchgeführt werden. Anstelle der Ausgangsstoffe IX kann man auch die Reaktionsgemische ihrer Herstellung, z.B. aus NO und Halogen, insbesondere Brom, verwenden.

Man kann die Ausgangsstoffe in stöchiometrischer Menge oder jede der Komponenten jeweils im Überschuß zur anderen umsetzen, vorzugsweise in einem Verhältnis von 1 bis 3, insbesondere von 1,05 bis 1,2 Mol Alkalinitrit oder Alkylnitrit, vorzugsweise 1 bis 3, insbesondere von 1,05 bis 1,2 Mol $HBF_4$ oder von 1 bis 3, insbesondere 1,05 bis 1,5 Mol Ausgangsstoff IX je Mol Stoff 1a. Man verwendet 1 bis 5, vorzugsweise 2 bis 4 Mol Ausgangsstoff III je Mol Stoff 1a. Die Umsetzung 1a) bzw. 1b) wird bei einer Temperatur von -50 bis +50°C, im Falle des Verfahrens 1a) zweckmäßig von -30 bis +40, insbesondere von -20 bis +25°C, im Falle des Verfahrens 1b) zweckmäßig von -25 bis +40, insbesondere von -25 bis 0°C, mit Unterdruck oder Überdruck oder drucklos, diskontinuierlich oder kontinuierlich durchgeführt. Man verwendet für Verfahren 1a) und 1b) Lösungsmittel. Wasser wird ganz oder teilweise in Gestalt von Lösungen der Säuren III und vorteilhaft in Gestalt von Lösungen des Alkalinitrits der Reaktion zugeführt. Als organische Lösungsmittel kommen im allgemeinen je nach Verfahren 1a oder 1b in Frage:

Halogenkohlenwasserstoffe (bevorzugt 1b), insbesondere Chlorkohlenwasserstoffe; Ether (bevorzugt 1a); Alkanole und Cycloalkanole (bevorzugt 1a); Sulfoxide und Sulfone; Ester (bevorzugt in Abwesenheit zusätzlicher Wassermengen); Carbonsäuren (bevorzugt 1a) mit 1 bis 6 Kohlenstoffatomen; und entsprechende Gemische. Zweckmäßig verwendet man das organische Lösungsmittel und/oder zusätzliches Wasser in einer Menge von 50 bis 5000 Gew.%, vorzugsweise von 100 bis 1000 Gew.%, bezogen auf Stoff 1a. Man kann einen Teil des Lösungsmittels oder die Gesamtmenge auch in Gestalt der entsprechenden Lösung der Ausgangsstoffe, z.B. der Tetrafluorborsäure, einsetzen. Ausgangsstoff III wird in Form einer 10- bis 50-, vorzugsweise 30 bis 70-gewichtsprozentigen Lösung verwendet. Die Reaktionszeit beträgt zweckmäßig von 0,2 bis 5 Stunden. In einer bevorzugten Ausführungsform arbeitet man mit Nitrosylbromid im Eintopfverfahren. Zunächst wird das in einem geeigneten Lösungsmittel, z.B. einem der vorgenannten, vorgelegte Brom durch Einleiten von NO bei +10°C bis -40°C, vorzugsweise -10°C bis -20°C, in NOBr überführt und anschließend das in einem vorgenannten Lösungsmittel gelöste 2-Aminopyrazin 1a bei der gleichen Temperatur zudosiert.

Der Stoff VIII kann mit Kupfercyanid allein in stöchiometrischer Menge oder zweckmäßig im Überschuß, vorteilhaft in einer Menge von 1 bis 2, bevorzugt 1,05 bis 1,5 Mol Kupfercyanid je Mol Ausgangsstoff VIII umgesetzt werden. Anstelle eines Anteils an Kupfercyanid können Alkali- und/ der Erdalkalicyanide, zweckmäßig Lithiumcyanid, Kalziumcyanid, Bariumcyanid, bevorzugt Natriumcyanid oder Kaliumcyanid, verwendet werden, zweckmäßig in Mengen von 1 bis 3 Mol, vorzugsweise 1,1 bis 1,5 Mol Alkali- und/oder Erdalkalicyanide je Mol Ausgangsstoff VIII. Vorteilhaft sind Gemische von 10 bis 50, bevorzugt 15 bis 25 Mol % Kupfercyanid, bezogen auf 100 Mol % aller Cyanide. Die Umsetzung wird bei einer Temperatur von 50 bis 200°C, vorzugsweise 120 bis 160°C, drucklos, unter Uberdruck oder Unterdruck, kontinuierlich oder diskontinuierlich durchgeführt. Man verwendet zweckmäßig unter den Reaktionsbedingungen inerte heterocyclische Lösungsmittel, zweckmäßig in einer Menge von 100 bis 5000, bevorzugt 200 bis 1000 Gew.% Lösungsmittel, bezogen auf Stoff 1a. Die Reaktionszeit beträgt 1 bis 12 Stunden.

Durch diese elegante Arbeitsweise in 3 Stufen, nämlich die Herstellung des Ausgangsstoffs II und aus diesem des Endstoffs Ia und aus diesem des Endstoffs Ic nach den vorgenannten Arbeitsweisen, werden zahlreiche Synthesewege auf den vorgenannten Industriegebieten, z.B. auch für Folsäureverbindungen, überraschend ermöglicht bzw. wirtschaftlicher und einfacher.

In den folgenden Beispielen bezieht sich die Ausbeute auf umgesetzten Ausgangsstoff II.

**Verfahren a) mit Chlor- und Bromwasserstoff**

**Beispiele 1-4**

Die in der Tabelle 1 aufgeführten α-Iminodiacetonitrile wurden in einem Lösungsmittel gelöst oder aufgeschlämmt und in einem mit Ankerrührer ausgestatteten Autoklaven mit Halogenwasserstoff bei 1 bis 5° innerhalb 1 Stunde begast. Anschließend erwärmte man das Gemisch auf die angegebene Maximaltemperatur und ließ das Gemisch nachreagieren. Nach dem Entspannen wurde das Gemisch mit Natronlauge auf pH 5 gestellt, die organische Phase abgetrennt und die wäßrige erschöpfend mit Methylenchlorid extrahiert. Die vereinigten organischen Phasen wurden destilliert.

Einzelheiten der Synthese führt die Tabelle 1 auf, die wichtigsten physikalischen Bigenschaften der isolierten Pyrazine fassen die Tabelle 5 und 6 zusammen.

Tabelle 1: Cyclisierung von Iminodiacetonitrilen II nach Verfahren a)

| Bsp. | Menge in g | Iminodiacetonitril II $R^1$ | $R^2$ | Lösungsmittel | g/Mol II | Halogenwasserstoff | Mol/Mol II | Temp. °C max. | Druck max. bar | Zeit bei T.max. (h) | Ausbeute (% d.Th.) der Endstoffe Ia | X | Ib | Ia + Ib |
|---|---|---|---|---|---|---|---|---|---|---|---|---|---|---|
| 1 | 95 | H | H | $CH_2ClCH_2Cl$ | 650 | HBr | 3,2 | 80 | 7,2 | 6 | 45 | Br | 1 | 46 |
| 2 | 123 | $CH_3$ | $CH_3$ | $CH_2ClCH_2Cl$ | 650 | HBr | 3,2 | 50 | 1,2 | 6 | 63 | Br | 4 | 67 |
| 3 | 123 | $CH_3$ | $CH_3$ | $CH_2Cl_2$ | 800 | HBr<br>HCl | 1,0<br>2,4 | 50 | 2,2 | 6 | 20 | Cl<br>Br | 10<br>3 | 33 |
| 4 | 68,5 | $C_2H_5$ | $CH_3$ | $CH_2Cl-CH_2Cl$ | 650 | HBr | 3,2 | 50 | 1 | 6 | Iaa 32<br>Iab 25 | Br | 3 | 60 |

**Verfahren b) mit Alkohol**

**Beispiel 5-18**

Die in der Tabelle 2 aufgeführten substituierten α-Iminodiacetonitrile II wurden in dem angegebenen Lösungsmittel gelöst und mit dem angegebenen Alkohol IV versetzt. In diese Lösung wurden nun bei 25°C (40°C; im Autoklaven bei 1 bar: Beispiel 10) mindestens 2,6 Mol HCl pro Mol Ausgangsstoff II während 60 Minuten eingegast und das Gemisch während 60 Minuten auf die Reaktionstemperatur (T max) gebracht. Durch potentiometrische-Titratioa der überschüssigen Salzsäuremenge wurde die Reaktion verfolgt. War der Umsatz konstant, wurde das Gemisch mit 20 gew.-%iger Natronlauge auf pH 5 gestellt, die organische Phase abgetrennt und die wäßrige Phase mehrmals mit Isobutanol extrahiert. Die vereinigten organischen Phasen wurden im Vakuum bei 60°C eingeengt und der Rest bei 0,5 mbar und einer Sumpftemperatur bis 160°C vom nicht flüchtigen Rückstand befreit. Die leicht kristallisierenden Destillate wurden aus Diisopropylether umkristallisiert und die Mutterlauge bis zur Lösungsmittelfreiheit eingeengt. Die zurückbleibenden Öle wurden gaschromatographisch analysiert und fraktioniert destilliert.

Die Aufarbeitung des 2-Amino-3,5-dimethylpyrazins (Beispiel 7-10) im Gemisch mit 2-Isobutoxi-3,5-dimethyipyrazin erioigte durch Extrakrion mi, Wasser und Nachreinigung der 50 gew.-%iger wäßrigen Lösung mit Wasserdampf (2-Isobutoxi-3,5-dimethylpyrazin ist gut wasserdampfflüchtig).

Einzelheiten der Synthese führt Tabelle 2 auf, die wichtigsten physikalischen Eigenschaften der isolierten Pyrazine fassen die Tabellen 5 und 6 zusammen.

**Tabelle 2**: Verfahren b) mit Alkohol/Cyclosierung von

α-Iminodiacetonitrilen II zu 2-Amino-3,5-Diakylpyrazinen Ia

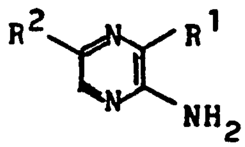

2-Alkoxi-3,5-Dialkylpyrazinen Ib2

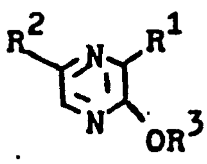

| Bsp. | Menge in g | α-Iminodiacetonitril II $R^1$ | $R^2$ | Lösungsmittel | $R^3OH$ g/Mol Ausgangsstoff II | | Mol/Mol Ausgangsstoff II | HCl Mol/Mol Ausgangsstoff II |
|---|---|---|---|---|---|---|---|---|
| 5 | 123 | $CH_3$ | $CH_3$ | Dioxan | 292 | n-Propanol | 1,0 | 2,6 |
| 6 | " | " | " | " | 241 | n-Butanol | 1,5 | 3,2 |
| 7 | " | " | " | " | 241 | i-Butanol | 1,5 | 3,2 |
| 8 | 369 | " | " | Diisopropylether | 148 | " | 2,0 | 3,2 |
| 9 | 123 | " | " | - | - | " | 4,8 | 3,2 |
| 10 | " | " | " | 1,2-Dichlorethan | 650 | " | 1,0 | 3,2 |
| 11 | " | " | " | - | - | 3-Methylbutanol | 4,0 | 3,2 |
| 12 | " | " | " | Dioxan | 176 | 2-Methylbutanol | 2,0 | 3,2 |
| 13 | " | " | " | " | 222 | 2-Ethylhexanol | 1,0 | 3,2 |

Tabelle 2: Fortsetzung

| Bsp. | Temperatur (°C max.) | Zeit bei T. max (h) | Ausbeuten (% d.Th.) an Endstoff Ia | Ib2 | Ia + Ib2 |
|---|---|---|---|---|---|
| 5 | 40 | 6 | 28 | 11 | 39 |
| 6 | 40 | 3 | 38 | 17 | 55 |
| 7 | 40 | 5,5 | 49 | 28 | 77 |
| 8 | 50 | 3,5 | 54 | 29 | 83 |
| 9 | 50 | 7 | 48 | 28 | 76 |
| 10 | 50 | 6 | 59 | 29 | 88 |
| 11 | 40 | 5,5 | 30 | 22 | 52 |
| 12 | 40 | 4 | 54 | 22 | 76 |
| 13 | 40 | 4 | 46 | 20 | 66 |

| Bsp. Nr. | Menge in g | -Iminodiaceto-nitril II $R^1$ | $R^2$ | Lösungsmittel | g/Mol Ausgangsstoff II | $R^3OH$ | Mol/Mol Ausgangsstoff II | HCl Mol/Mol Ausgangsstoff II |
|---|---|---|---|---|---|---|---|---|
| 14 | 123 | $CH_3$ | $CH_3$ | Dioxan | 191 | Chlorethanol | 1,0 | 2,6 |
| 15 | " | " | " | - | - | Glykolsäure-n--butylester | 3,0 | 3,2 |
| 16 | " | " | " | Dioxan | 217 | Ethoxyethanol | 1,5 | 3,2 |
| 17 | 96 | $C_2H_5$ | " | " | 163 | i-Butanol | 1,5 | 3,2 |
| 18 | 151 | " | $C_2H_5$ | " | 204 | " | 2,0 | 5,1 |

| Bsp. | Temperatur (°C max.) | Zeit bei T. max (h) | Ausbeuten (% d.Th.) an Endstoff Ia | Ib2 | Ia + Ib2 |
|---|---|---|---|---|---|
| 14 | 50 | 6,5 | 55 | 10 | 65 |
| 15 | 40 | 5 | 44 | 1 | 44-45 |
| 16 | 40 | 5 | 57 | 21 | 78 |
| 17 | 40 | 6,5 | 44[1)] | 21 | 65 |
| 18 | 40 | 4 | 43 | 19 | 62 |

1) röntgenographische Strukturbestimmung

**Verfahren b) mit Thiolen**

**Beispiele 19-35**

Die in der Tabelle 3 aufgeführten substituierten -Iminodiacetonitrile II wurden in dem angegebenen Lösungsmittel gelöst und mit dem angegebenen Mercaptan versetzt. In diese Lösung wurde während 60 Minuten (im Autoklaven: Beispiel 30) HCl eingegast. In den Beispielen 25 und 26 wurde ein Teil HCl durch Methansulfonsäure bzw. Bromwasserstoff ersetzt. Anschließend wurde das Gemisch während 60 Minuten auf Reaktionstemperatur (T max) gebracht und unter heftigem Rühren (meist pastenförmige Gemische) umgesetzt. Durch poteniometrische Titration der überschüssigen Säure oder dünnschichtchromatographisch wurde der Fortgang der Reaktion verfolgt. War der Umsatz konstant, wurde das Gemisch mit 20 gew.-%iger Natronlauge auf pH 8,0 eingestellt, die organische Phase abgetrennt und die wäßrige Phase merhrmals mit Isobutanol extrahiert. Die vereinigten organischen Phasen wurden im Vakuum bei 60°C eingeengt und der Rest bei 0,5 mbar und einer Sumpftemperatur bis 160°C vom nicht flüchtigen Rüchstand befreit. Die meist gut kristallisierenden Destillate wurden umkristallisiert oder durch fraktionsierte Destillation gereinigt. Die Destillate wurden gaschromatographisch analysiert. Einzelheiten der Synthese zeigt Tabelle 3, die wichtigsten physikalischen Eingenschaften der isolierten Phyrazine I sind in Tabelle 5 und 6 zusammengefaßt.

**Tabelle 3**: Verfahren b) mit Thiolen/Cyclisierung von

α-Iminodiacetonidtrilen II zu
2-Amino-pyrazinen Ia
2-Alkylmercapto-pyrazinen (Y=S) (Ib1)
2-Alkoxipyrazinen (Y=O) (Ib2)
2-Halogen-3,5-diakylpyrazinen (Ib3)

| Bsp. | Menge in g | α-Iminodiacetonitril II $R^1$ | $R^2$ | Lösungsmittel | g/Mol Ausgangsstoff II | $R^3SH$ | Mol/Mol Ausgangsstoff II | HCl Mol/Mol Ausgangsstoff II |
|---|---|---|---|---|---|---|---|---|
| 19 | 65,4 | $CH_3$ | H | Dioxan | 400 | n-$C_4H_9SH$ | 0,5 | 2,9 |
| 20 | 75,5 | i-$C_4H_9$ | H | Dichlorethan | 650 | " | " | 3,2 |
| 21 | 123 | $C_2H_5$ | H | Dioxan | 307 | " | " | 3,3 |
| 22 | 43 | Phenyl | H | " | 460 | " | " | 3,5 |
| 23 | 123 | $CH_3$ | $CH_3$ | " | 321 | $C_2H_5SH$ | " | 3,0 |
| 24 | 123 | " | " | " | 307 | n-$C_4H_9SH$ | " | 2,9 |
| 25 | 123 | " | " | " | " | " | " | 1[2)] |
| 26 | 123 | " | " | " | " | " | " | 1,1[3)] |

**Tabelle 3**: Fortsetzung

| Bsp. | Temperatur °C max. | Zeit T. max. (h) | Ausbeuten (% d.Th.) an Endstoff Ia | Ibl | Summe Pyrazine I | Umsatz (%) |
|---|---|---|---|---|---|---|
| 19 | 30 | 5 | Iaa 28[1)] | 6 | 37 | 80 |
| | | | Iab 3 | | | |
| 20 | 50 | 5 | 74 | 2 | 76 | |
| 21 | 40 | 5 | 44 | 4 | 48 | |
| 22 | 40 | 5,5 | Iaa 18 | 3 | 45 | |
| | | | Iab 24 | | | |
| 23 | 40 | 9 | 64 | 8 | 72 | |
| 24 | 60 | 5 | 70 | 7 | 77 | |
| 25 | 50 | 13 | 35 | 4 | 39 | 85 |
| 26 | 50 | 8 | 32 | 8 | 44 | |
| | | | | 4 Ib3 (X=Br) | | |

| Bsp. | Menge in g | -Iminodiacetonitril II $R^1$ | $R^2$ | Lösungsmittel | g/Mol Ausgangsstoff II | $R^3SH$ | Mol/Mol Ausgangsstoff II | HCl Mol/Mol Ausgangsstoff II |
|---|---|---|---|---|---|---|---|---|
| 27 | 123 | $CH_3$ | $CH_3$ | Dioxan | 284 | n-$C_4H_9SH$ | 0,75 | 3,2 |
| 28 | 123 | " | " | i-Butanol | 307 | i-$C_4H_9SH$ | 0,5 | 3,2 |
| 29 | 123 | " | " | " | 410 | Octanthiol | 0,25 | 3,2 |
| 30 | 123 | " | " | 1,2-Dichlorethan | 650 | n-$C_4H_9SH$ | 0,5 | 3,2 |
| 31 | 137 | $C_2H_5$ | " | Dioxan | 307 | i-$C_4H_9SH$ | 0,5 | 3,2 |
| 32 | 226,5 | i-$C_3H_7$ | " | " | " | n-$C_4H_9SH$ | 0,5 | 3,5 |
| 33 | 165,6 | 2-Ethylpentyl | " | " | " | " | " | 3,3 |
| 34 | 59,7 | Benzyl | " | " | 675 | " | " | 5,3 |
| 35 | 18,5 | Phenyl | " | " | 400 | " | " | 3,2 |

**Tabelle 3**: Fortsetzung

| Bsp. | Temperatur °C max. | Zeit T. max. (h) | Ausbeuten (% d.Th.) an Endstoff Ia | Ib1 | Summe Pyrazine I | Umsatz (%) |
|---|---|---|---|---|---|---|
| 27 | 40 | 5,5 | 63 | 13 | 76 | |
| 28 | 50 | 8 | 55 | Ib2 32 | 87 | |
| 29 | 60 | 3 | 44 | 30 | 74 | |
| 30 | 50 | 6 | 60 | 5+Ib3 (X=Cl)1 | 66 | |
| 31 | 40 | 6 | Iaa 66<br>Iab 4 | | | |
| 32 | 40 | 9 | 40 | | | 90 |
| 33 | 40 | 5 | 50 | | | 70 |
| 34 | 45 | 6 | 42 1) | | | |
| 35 | 40 | 6 | Iaa 15<br>Iab 35 | | | |

1) röntgenographische Strukturbestimmung
2) + 2,2 Mol $CH_3SO_3H$
3) + 1 Mol HBr

**Verfahren c**

**Beispiele 36-44**

Die in Tabelle 4 aufgeführten substituierten α-Iminodiacetonitrile II wurden in Methanol zu einer 15 gew.%igen Lösung gelöst und bei 22°C mit 0,5 Mol Hilfsbasen pro Mol Ausgangsstoff II versetzt. Der Fortang der Reaktion wurde dünnschichtchromatographisch verfolgt. Am Ende der Reaktion wurde mit konzentrierter Salzsäure auf pH 5,0 gestellt und das Lösungsmittel zusammen mit freigesetzter Blausäure im schwachen Vakuum abgetrennt. Der Rückstand wurde pro Mol eingesetztem Dinitril II mit 100 g Wasser versetzt und mit Methylenchlorid extrahiert. Die Extrakte wurden eingeengt und die Rückstände destilliert. Die Pyrazingemische destillierten bei einem Kp von 100 bis 120°C (0,5 mbar) über. Die Alkoxypyrazine waren wesentlich leichter flüchtig, so daß ihre Abtrennung keine Schwierigkeiten bedeutete, Die Destillate wurden GC-analysiert und die-Hauptkpmponenten durch fraktionierte Destillation oder durch Umkristallisation aus Diisopropylether, gegebenenfalls unter Hexanzugabe, umkristallisiert. Die reinen Pyrazine wurden durch Elementaranalyse, NMRund UV-Spektrum charakterisiert. Einzelheiten der Synthese bringt Tabelle 4. Die wichtigsten physikalischen Eigenschaften der rein isolierten Pyrazinderivate fassen die Tabelle 5 und 6 zusammen.

**Tabelle 4**: Verfahren c) /Cycloiseierung von α-Iminodiacetonitrilen II

zu 2-Amino-pyrazinen Ia
2-Alkoxi-pyrazinen Ib
2-Amino-6-alkoxipyrazinen Ic

| Bsp. | Menge in g | α-Iminodiacetonitril II $R^1$ | $R^2$ | $R^3OH$ | Hilfsbase | Temperatur °C | Zeit (Tage) | Ausbeute (% d.Th.) Ia | Ib | Ic |
|---|---|---|---|---|---|---|---|---|---|---|
| 36 | 123 | $CH_3$ | $CH_3$ | $CH_3OH$ | $NaOCH_3$ | 40 | 3 | 48 | | 24 |
| 37 | 137 | $C_2H_5$ | $CH_3$ | " | " | " | 5 | Iaa 20<br>Iab 14 | Iba 10<br>Ibb 3 | - |
| 38 | 15,3 | $CH_3OCH_2$ | " | " | $NaOCH_3$/KCN (1:1) | 22 | 4 | | | 40 |
| 39 | 16,7 | $C_2H_5OCH_2$ | " | " | $NaOCH_3$ | 22 | 4 | | | 44 |
| 40 | 30,2 | $CH(CH_3)_2$ | " | " | " | 40 | 6 | | | 22 |
| 41 | 10 | 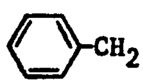 | " | " | " | 22 | 4 | Iaa 20<br>Iab 11 | Iba 16<br>Ibb 6 | - |
| 42 | 15,1 | $C_2H_5$ | $C_2H_5$ | " | " | 40 | 6 | 30 | | - |
| 43 | 18,3 | $CH_3OCH_2$ | $CH_3OCH_2$ | " | $LiOCH_3$ | 22 | 3 | | | 49 1) |
| 44 | 18,3 | " | " | " | NaOH | " | 2 | | | 42 |

1) röntgenographische Strukturbestimmung

**Tabelle 5**

| Bsp. | Formel | Fp (°C) | Reinheit Gew.% | UV-Spektren in Methanol max.1 | $E_1$ | max.2 | $E_2$ |
|---|---|---|---|---|---|---|---|
| 1 | 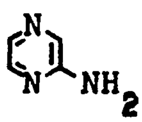 | 115-117 | 99,5 | 232 | 10415 | 316 | 6168 |
| 19 | 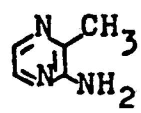 | 166-168 | 100 | 233 | 10360 | 316 | 6209 |
| 21 | 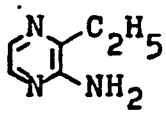 | 49- 50 | 99,2 | 234 | 9962 | 319 | 6288 |
| 20 | 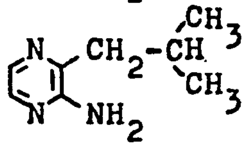 | 80- 81 | 98,7 | 270 | 8449 | 335 | 7989 |
| 22 | 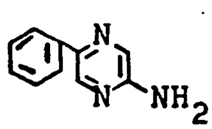 | 140- 42 | 98,9 | 215 | 9506 | | |
| 23 | 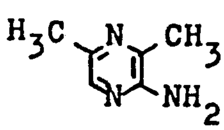 | 93- 95 | 99,6 | 234 | 11107 | 324 | 6210 |

**Tabelle 5**: Forsetzung

| Bsp. | Formel | Fp (°C) | Reinheit | UV-Spektren in Methanol | | | |
|---|---|---|---|---|---|---|---|
| | | | Gew.% | max.$_1$ | $E_1$ | max.$_2$ | $E_2$ |
| 31 | 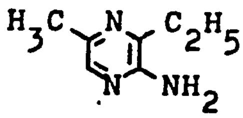 | 49- 52 | 95,3 | 235 | 10605 | 325 | 6161 |
| 32 | 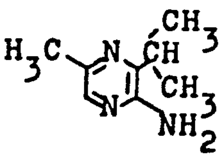 | 56- 60 | 97,5 | 234 | 10828 | 323 | 6487 |
| 33 | 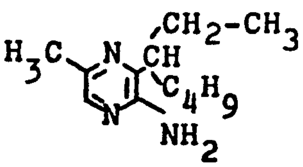 | 74- 75 | 97 | 235 | 10787 | 327 | 6610 |
| 34 | 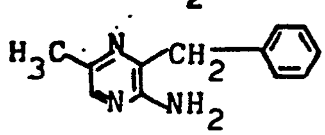 | $n_D^{20}$ = 1.6125 | 97 | 234 | 10233 | 327 | 6427 |
| 35 | 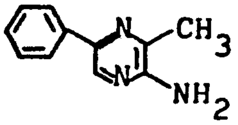 | 120-130 | 98 | 275 | 15231 | 334 | 10106 |
| 18 | 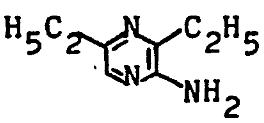 | 47-48 | 99,7 | 235 | 11677 | 324 | 6632 |
| 38 | 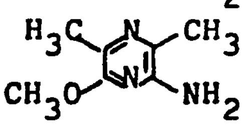 | 94-95 | 97,2 | 236 | 11030 | 328 | 10528 |
| 43 | 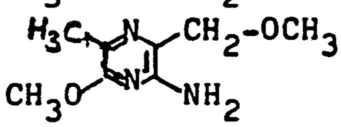 | 52-55 | 96,3 | 243 | 11329 | 326 | 9719 |

**Tabelle 5**: Fortsetzung

| Bsp. | Formel | Kp (°C/mb) | $n_D^{20}$ | Reinheit Gew.% | UV-Spektren in Methanol max.1 | $E_1$ | max.2 | $E_2$ |
|---|---|---|---|---|---|---|---|---|
| 36 | 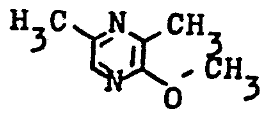 | 74/20 | 1.5041 | 99,5 | | | | |
| 5 | 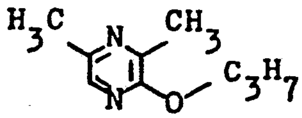 | 66/0,4 | | | | | | |
| 7 | 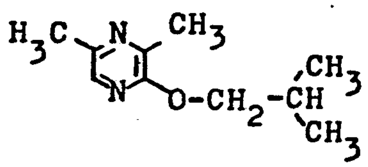 | | 1.4840 | 98,8 | 216 | 11641 | 300 | 6899 |
| 12 | 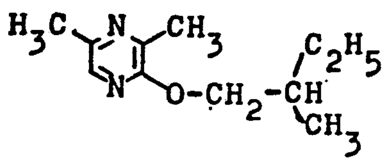 | | 1.4850 | 98,7 | 216 | 11485 | 300 | 6939 |
| 13 | 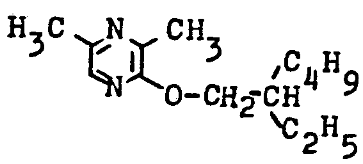 | | 1.4826 | 97 | 216 | 11418 | 300 | 6752 |
| 16 | 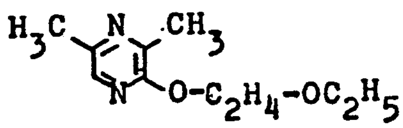 | | 1.4890 | 96,8 | 215 | 10772 | 299 | 6590 |
| 28 | 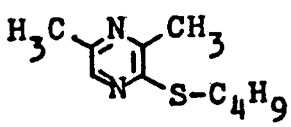 | | 1.5421 | 97,3 | 248 | 11640 | 322 | 7105 |

**Tabelle 6**

| Bsp. | Formel | Kp (°C/mb) | $n_D^{20}$/Fp (°C) | Reinheit Gew.% | UV-Spektren in Methanol λ max.1 | $E_1$ | λ max.2 | $E_2$ |
|---|---|---|---|---|---|---|---|---|
| 17 | 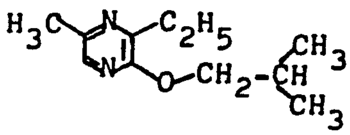 | 63-65/ 0,3 | | | | | | |
| 3 | 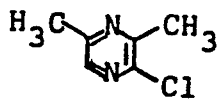 | 87/30 | 1.5269/ 9-10°C | 99 | 211 | 9419 | 279 | 6954 |
| 2 | 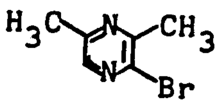 | 55/0,3 | 1.5590/ 7-8°C | 99 | | | | |

**Patentansprüche**

1. Verfahren zur Herstellung von Gemischen von 2-Aminopyrazinen der Formel Ia

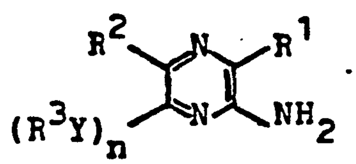

Ia

und von Fyrazinen der Formel Ib

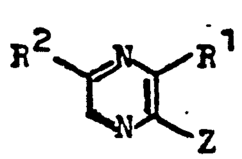

Ib,

worin die einzelnen Reste $R^1$ und $R^2$ gleich oder verschieden sein können und jeweils ein Wasserstoffatom einen Alkylrest mit 1 bis 20 Kohlenstoffatomen der im Falle des Verfahrens c) durch Alkoxyund/oder Alkylthiogruppen mit 1 bis 4 Kohlenstoffatomen im Falle des Verfahrens a) und b) durch Halogenatome substituiert sein kann einen Alkenylrest mit 2 bis 20 Kohlenstoffatomen einen Cycloalkylrest mit 5 bis 8 Kohlenstoffatomen einen Alkylarylrest oder Aralkylrest mit 7 bis 12 Kohlenstoffatomen oder einen Phenylrest bedeuten $R^3$ im Falle des Verfahrens b) einen ggf. durch Halogenatome Carbalkoxygruppen mit 2 bis 4 Kohlenstoffatomen substituierten Alkylrsst mit 1 bis 20 Kohlenstoffatomen bedeutet im Falle des Verfahrens c) einen Alkylrest mit 1 bis 8 Kohlenstoffatomen bezeichnet Y fur ein Sauerstoffatom oder Schwefelatom steht 2 ein Bromatom oder Chloratom X oder den Rest $YR^3$, worin Y und Ra die vorgenannte Bedeutung besitzen bedeutet und n O oder wenn die Umsetzung c) durchgeführt wird ggf. 1 bezeichnet durch Cyclisierung von Dinitrilverbindungen dadurch gekennzeichnet,
daß man α-Imino-diacetonitrile der Formel II

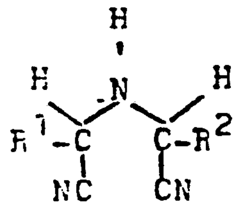

II,

zur Herstellung der Verbindungen Ia und Ib

a) für den Fall daß 2 ein Brom- oder Chloratom X bedeutet mit Brom- oder Chlorwasserstoffen der Formel III

H - X III

oder

b) für den Fall daß 2 ein Brom- oder Chloratom X oder den Rest $YR^3$ bedeutet wobei $R3^3$ mehr als 1 C-Atom aufweist, mit Alkoholen und/oder Thioalkoholen der Formel (V

$HYR^3$ IV

und Brom- oder Chlorwasserstoffen der Formel III

H - X III

oder

c) für den Fall daß Z den Rest $YR^3$ bedeutet mit Alkoholen und/oder Thioalkoholen der Formel (V

$HYR^3$ IV

in Gegenwart von Alkali- und/oder Erdalkaliverbindungen umsetzt

2. 2-Aminopyrazine der Formel Ia

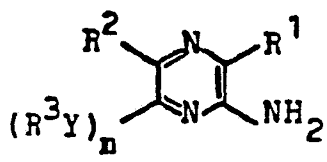

Ia,

worin die einzelnen Reste $R^1$ und $R^2$ verschieden sind und ein Wasserstoffatom einen Alkylrest mit 1 bis 20 Kohlenstoffatomen einen Alkenylrest mit 2 bis 20 Kohlenstoffatomen einen durch Alkoxy-und/ oder Alkylthiogruppen mit 1 bis 4 Kohlenstoffatomen oder einen durch Halogenatome substituierten Alkylrest mit 1 bis 20 Kohlenstoffatomen einen Cycloalkylrest mit 5 bis 8 Kohlenstoffatomen einen Alkylarylrest oder Aralkylrest mit 7 bis 12 Kohlenstoffatomen oder einen Phenylrest bedeuten $R^3$ einen ggf. durch Halogenatome oder Carbalkoxygruppen mit 2 bis 4 Kohlenstoffatomen substituierten Alkylrest mit 1 bis 20 Kohlenstoffatomen bezeichnet Y fur ein Sauerstoffatom oder Schwefelatom steht und n 0 oder 1 bezeichnet wobei wenn $R^1$ und $R^2$ jeweils ein Wasserstoffatom einen Methyl- Ethyl- einen über Stickstoffatome oder Sauerstoffatome substituierten Methylrest oder einen Phenylrest bedeuten dann n 1 bezeichnet.

## Claims

1. A process for the preparation of a mixture of a 2-aminopyrazine of the formula Ia

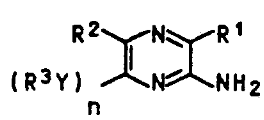

Ia

with a pyrazine of the formula Ib

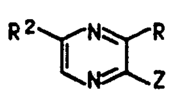

Ib

where the individual radicals $R^1$ and $R^2$ may be identical or different and are each hydrogen or alkyl of 1 to 20 carbon atoms which may be substituted by alkoxy and/or alkylthio each of 1 to 4 carbon atoms in the case of reaction (c) or by halogen in the case of reactions (al and (b) or are each alkenyl of 2 to 20 carbon atoms cycloalkyl of 5 to B carbon atoms alkylaryl or aralkyl of 7 to 12 carbon atoms or phenyl Ra is in the case of reaction (b) alkyl of 1 to 20 carbon atoms which is unsubstituted or substituted by halogen or carbalkoxy of 2 to 4 carbon atoms or is in the case of reaction (c) alkyl of 1 to B carbon atoms Y is oxygen or sulfur 2 is a bromine or chlorine atom X or a radical $YR^3$ where Y and $R^3$ have the aforementioned meanings and n is 0 or when reaction (c) is carried out may be 1 by cyclization of a dinitrile compound wherein for the preparation of compounds Ia and Ib an α-iminodiacetonitrile of the formula II

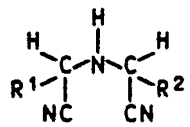

II

is reacted
(a) where 2 is a bromine or chlorine atom X with hydrogen bromide or hydrogen chloride of the formula III
H - X III
(b) where 2 is a bromine or chlorine atom X or a radical $YR^3$ $R^3$ having more than 1 carbon atom with an alcohol and/or a thioalcohol of the formula IV
$HYR^3$ IV
and hydrogen bromide oder hydrogen chloride of the formula III
H - X
or
(c) where 2 is a radical $YR^3$ with an alcohol and/or thioalcohol of the formula IV
$HYR^3$ IV,
in the presence of an alkali metal compound and/or an alkaline earth metal compound.

2. A 2-aminopyrazine of the formula Ia

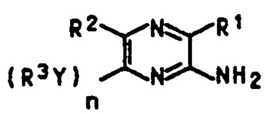

Ia,

where the individual radicals $R^1$ and $R^2$ may be identical or different and are each hydrogen alkyl of 1 to 20 carbon atoms alkenyl of 2 to 20 carbon atoms alkyl of 1 to 20 carbon atoms substituted by alkoxy and/or alkylthio each of 1 to 4 carbon atoms or by halogen cycloalkyl of 5 to B carbon atoms alkylaryl or aralkyl of 7 to 12 carbon atoms or phenyl Ra is alkyl of 1 to 20 carbon atoms which is unsubstituted or substituted by halogen or carbalkoxy of 2 to 4 carbon atoms Y is oxygen or sulfur and n is 0 or 1 and if $R^1$ and $R^2$ are each hydrogen methyl or ethyl or a methyl radical which is substituted via nitrogen atoms or oxygen atoms, or are each phenyl, then n is 1.

**Revendications**

1. Procédé de préparation de mélanges de 2-aminopyrazines de formule Ia

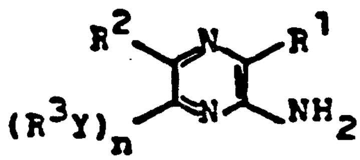

Ia

et de pyrazines de formule Ib

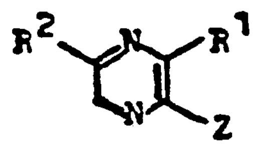

Ib,

dans lesquelles les différents radicaux R' et $R^2$ peuvent être identiques ou dissemblables et représentent chacun un atome d'hydrogène, un radical alkyle à 1- 20 atomes de carbone qui peut être substitué, dans le cas du procédé c), par des groupes alcoxy- et/ou alkylthio à 1 - 4 atomes de carbone et, dans le cas des procédés a) et b), par des atomes d'halogène, un radical alcényle à 2 - 20 atomes de carbone, un radical cycloalkyle à 5 - 8 atomes de carbone, un radical alkylaryle ou aralkyle à 7 - 12 atomes de carbone ou un radical phényle, $R^3$ représente, dans le cas du procédé b), un radical alkyle à 1-20 atomes de carbone éventuellement substitué par des atomes d'halogènes, des groupes carbalcoxy a 2 - 4 atomes de carbone et, dans le cas du procédé c), un radical alkyle à 1 - 8 atomes de carbone, Y est mis pour un atome d'oxygène ou un atome de soufre, Z est un atome de brome, un atome de chlore X ou le radical $YR^3$, Y et $R^3$ ayant les significations données ci-dessus et n est mis pour O ou éventuellement, au cas où la réaction c) est effectuée, pour 1, par cyclisation de composés dinitrile, caractérisé en ce que, pour la préparation des composés Ia et Ib, on fait réagir des α-iminodiacétonitriles de formule II

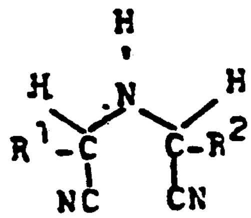

II,

a) dans le cas où Z représente un atome de brome ou de chlore X, avec un acide bromhydrique ou chlorhydrique de formule III

H - X (III)

ou

b) dans le cas où Z représente un atome de brome ou de chlore X ou le radical $YR^3$, $R^3$ comportant plus de 1 atome de C, avec des alcools et/ou des thio-alcools de formule IV

$HYR^3$ (IV)

et un acide bromhydrique ou chlorhydrique de formule

H - X (III)

ou

c) dans le cas où Z représente le radical $YR^3$, avec des alcools et/ou des thio-alcools de formule IV

$HYR^3$ (IV)

en présence de composés alcalins et/ou alcalino-terreux.

2. 2-aminopyrazines de formule Ia

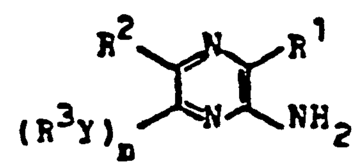

Ia,

dans laquelle les differents radicaux $R^1$ et $R^2$ sont dissemblables et représentent chacun un atome d'hydrogène, un radical alkyle à 1 - 20 atomes de carbone, un radical alcényle à 2 - 20 atomes de carbone, un radical alkyle à 1 - 20 atones de carbone substitué par des groupes alcoxy et/ou alkylthio à 1 - 4 atomes de carbone ou par des atomes d'halogène, un radical cycloalkyle à 5-8 atomes de carbone, un radical alkylaryle ou aralkyle à 7 - 12 atomes de carbone ou un radical phényle, $R^3$ représente un radical alkyle à 1 - 20 atomes de

carbone éventuellement substitué par des atomes d'halogène ou des groupes carbalcoxy à 2 - 4 atomes de carbone, Y est mis pour un atome d'oxygène ou un atome de soufre en n est égal à 0 ou 1, n étant égal à 1 lorsque $R^1$ et $R^2$ représentent respectivement un atome d'hydrogène, un radical méthyle, éthyle, ou méthyle substitué par l'intermédiaire d'atomes d'azote ou d'atomes d'oxygène, ou un radical phényle.